# EUROPÄISCHE PATENTSCHRIFT

(11) **EP 1 049 414 B1**
(45) Veröffentlichungstag und Bekanntmachung des Hinweises auf die Patenterteilung: **28.04.2004**
(21) Anmeldenummer: 99908809.9
(22) Anmeldetag: 21.01.1999
(51) Int. Cl.: A61B 17/80

(54) **RIEGEL ZUR VERBINDUNG EINER FRAKTUR DES BECKENS IM BEREICH DER SCHAMBEINFUGE**
PLATE FOR CONNECTING A PELVIC FRACTURE IN THE AREA OF A THE SYMPHYSIS PUBIS
PLAQUE POUR JOINDRE UNE FRACTURE DU BASSIN DANS LA REGION DU JOINT DU PUBIS

(30) Priorität: 22.01.1998 DE 19802229
(43) Veröffentlichungstag der Anmeldung: 08.11.2000
(73) Patentinhaber: Steinbeis GmbH & Co. für Technologietransfer, 78050 Villingen-Schwenningen (DE)
(72) Erfinder: ZEIDLER, Hubert, D-09221 Neukirchen (DE); PIERER, Wolfgang, D-09126 Chemnitz (DE)
(74) Vertreter: Heuckeroth, Volker, Dr.
(86) Internationale Anmeldenummer: PCT/EP1999/000374
(87) Internationale Veröffentlichungsnummer: WO 1999/037231

(56) Entgegenhaltungen:
- DE-A- 3 043 566
- DE-B- 2 603 087
- US-A- 4 454 876
- US-A- 4 573 458

## Beschreibung

Die Erfindung betrifft einen Riegel zur Verbindung einer Fraktur des Beckens im Bereich der Schambeinfuge, der beidseits der Fraktur an den zu verbindenden Beckenknochen-Abschnitten mittels Schrauben, Nägeln oder dgl. befestigbar ist.

Ein derartiger Riegel ist aus der DE-B-26 03 087 bekannt. Bei einer Fraktur der Knochenplatten oder Knochenplattenverbindungen des Beckens wird zur Osteosynthese, d.h. zur Unterstützung des Zusammenwachsens der gebrochenen Beckenknochen ein plattenförmiger Riegel am Becken befestigt, der die Frakturstelle in etwa quer zum Frakturverlauf überspannt und die beiden zu verbindenden Beckenknochen-Abschnitte aneinander fixiert.

Die herkömmlichen Riegel sind als einstückige starre Platten ausgebildet, die mittels Schrauben, Nägel oder dgl. an den Beckenknochen-Abschnitten befestigt werden.

Die als einstückige, starre, meist aus Edelstahl hergestellten Riegel sind jedoch nachteilig. Mit diesen herkömmlichen Platten werden die Beckenknochen-Abschnitte beidseits der Fraktur bezüglich aller Freiheitsgrade starr aneinander fixiert. Dabei wird nicht berücksichtigt, daß das Becken kein in sich starres Knochensystem darstellt. Das Becken wird vielmehr aus mehreren Beckenknochen-Abschnitten gebildet, die eine anatomisch vorgegebene Beweglichkeit relativ zueinander besitzen. Insbesondere sind die im Bereich des Steißbeines befindlichen unteren linken und rechten Beckenknochen, die Schambögen (Arci pubici), lediglich über einen Knorpelabschnitt, die Schambeinfuge (Symphysis pubica), miteinander verbunden. Die Knorpelverbindung zwischen den Schambögen wirkt als gelenkige Verbindung, die eine Relativbeweglichkeit der Schambögen bei verschiedenen Bewegungen des Menschen, bspw. beim Gehen, Liegen, Sitzen, zuläßt.

Bei einer Fraktur dieses Knorpelabschnittes, deren Verlauf zumeist vertikal ist, wird ein plattenförmiger Riegel an den beidseits des Knorpelabschnittes befindlichen Schambögen befestigt und überspannt dabei den Knorpelabschnitt quer zum Frakturverlauf, um den frakturierten Knorpelabschnitt verheilen zu lassen, indem die beiderseitigen Schambögen durch den Riegel zusammengehalten werden. Das Zusammenhalten der beiderseitigen Schambögen wird zwar durch einen herkömmlichen starren Riegel gewährleistet, jedoch verhindert der Riegel aufgrund seiner insgesamt starren Ausbildung auch alle anderen natürlichen Bewegungen der beiden Beckenknochen-Abschnitte relativ zueinander. Eine derartige starre Ausbildung weist auch die aus der obengenannten DE-B-26 03 087 bekannte Knochenplatte zur Fixation von Symphyse-Frakturen auf.

Diese bekannte Knochenplatte weist eine Platte auf, die aus körperverträglichem, sterilisierbarem thermoplastischem Kunststoff besteht und im wesentlichen überall gleiche Wandstärke besitzt. Die Platte ist in Form eines Kragens gebogen, der zwei rückseitig unverbundene Seitenteile und ein über die Seitenteile nach unten verlängertes Vorderteil mit einer mittigen Randeinbuchtung aufweist, wobei die Seitenteile unter entsprechender Auswölbung des Vorderteils mit ihrem rückwärtigen Rand leicht gegeneinander geschwenkt und im übrigen so weit eingerollt sind, daß sie im Bereich ihres rückwärtigen Randes etwa flächenparallel zur vorderseitigen Plattenfläche in deren Übergangsbereich vom Vorderteil zu den Seitenteilen verlaufen.

Eine Bewegung des Bruch-Patienten im Krankenbett, bspw. eine Drehbewegung des Patienten von der Rückenlage in die Seitenlage, führt bereits dazu, daß auf die beiden zuvor genannten unteren Beckenknochen-Abschnitte Drehmomente und Zugkräfte ausgeübt werden. Da der herkömmliche starre Riegel eine relative Bewegung dieser Beckenknochen-Abschnitte nicht zuläßt, müssen diese Kräfte von dem Riegel und den Schrauben bzw. Nägeln aufgenommen werden. Diese Kräfte sind derart hoch, daß sie zum Ausreißen der Schrauben bzw. Nägel führen können. Bei der Verwendung derartiger herkömmlicher Riegel muß der Patient somit vollkommen immobilisiert werden, um ein Ausreißen der Knochenschrauben bzw. -nägel zu vermeiden, was für den Patienten jedoch unangenehm ist.

Der Erfindung liegt somit die Aufgabe zugrunde, einen Riegel der eingangs genannten Art dahingehend weiterzubilden, daß sich der Beckenbruch-Patient im Krankenbett bewegen kann, ohne daß die Schrauben bzw. Nägel, mit denen der Riegel an den Beckenknochen befestigt ist, ausreißen können, wobei gleichzeitig gewährleistet ist, daß die Beckenfraktur sicher zusammengehalten wird und heilen kann.

Erfindungsgemäß wird die der Erfindung zugrundeliegende Aufgabe hinsichtlich des eingangs genannten Riegels dadurch gelöst, daß der Riegel aus zumindest zwei in Längsrichtung hintereinander angeordneten Teilen gebildet ist, wobei zur Verbindung der Teile an dem einen Teil ein erstes Eingriffsmittel vorgesehen ist, das mit einem an dem anderen Teil vorgesehenen zweiten Eingriffsmittel in Eingriff steht, derart, daß die beiden Teile in Längsrichtung und in Richtung quer zur Riegelebene aneinander gesichert sind, und daß die beiden Teile relativ zueinander eine begrenzte gelenkige Beweglichkeit aufweisen.

Die zweiteilige Ausgestaltung des erfindungsgemäßen Riegels hat gegenüber der herkömmlichen einstückigen starren Ausgestaltung den Vorteil, daß sie eine Relativbewegung der miteinander verbundenen Beckenknochen-Abschnitte ermöglicht. Dadurch, daß die beiden Teile über die beiden erfindungsgemäß vorgesehenen Eingriffsmittel so miteinander verbunden sind, daß die beiden Teile in Längsrichtung und in Richtung quer zur Riegelebene aneinander gesichert sind, kann die Fraktur quer zum Frakturverlauf sicher zusammengehalten werden. Somit wird gewährleistet, daß die Fraktur heilen kann, weil die Eingriffsmittel verhindern, daß sich die beiden durch den erfindungsgemäßen Riegel verbundenen Beckenknochen-Abschnitte beidseits der Fraktur auseinander bewegen können. Dadurch, daß erfindungsgemäß weiterhin vorgesehen ist, daß die beiden Teile relativ zueinander eine begrenzte gelenkige Beweglichkeit aufweisen, wird vorteilhaft bewirkt, daß die durch den erfindungsgemäßen Riegel miteinander verbundenen Beckenknochen-Abschnitte ihre natürliche, anatomisch vorgegebene begrenzte Relativ-Beweglichkeit beibehalten können. Dadurch wird bei einer Bewegung des Patienten wiederum vermieden, daß die Knochenschrauben bzw. -nägel aus den Beckenknochen-Abschnitten ausreißen. Der erfindungsgemäße Riegel weist dazu in einem begrenzten Winkelbereich Freiheitsgrade der Relativbewegung der beiden Teile auf. Die begrenzte Relativbewegung der beiden Teile kann bspw. dadurch erreicht werden, daß die Eingriffsmittel mit einem Spiel ineinandergreifen.

Somit wird die der Erfindung zugrunde liegende Aufgabe vollkommen gelöst.

In einer bevorzugten Ausgestaltung weisen die beiden Teile eine begrenzte gelenkige Beweglichkeit um eine quer zur Riegelebene verlaufende erste Schwenkachse auf.

Durch diese Maßnahme wird bewirkt, daß die beiden Teile in der Riegelebene relativ zueinander gegeneinander begrenzt verdreh- bzw. verschwenkbar sind. Dadurch wird der Vorteil erzielt, daß eine natürliche Beweglichkeit der Schambögen, etwa wenn der Patient beim Aufstehen ein Bein stärker belastet als das andere, aufrechterhalten bleibt.

In einer weiteren bevorzugten Ausgestaltung weisen die beiden Teile eine begrenzte gelenkige Beweglichkeit um eine in der Riegelebene quer zur Längsrichtung verlaufende zweite Schwenkachse auf.

Durch diese Maßnahme wird bewirkt, daß die beiden Teile aus der Riegelebene heraus relativ zueinander gegeneinander verdreh- bzw. verschwenkbar sind. Dadurch wird der Vorteil erzielt, daß eine Relativbeweglichkeit der Schambögen, etwa wenn sich der Patient im Bett dreht oder in eine Sitzstellung aufrichtet, ebenfalls aufrechterhalten wird.

In einer weiteren bevorzugten Ausgestaltung sind die beiden Teile relativ zueinander um ihre Längsachse gegensinnig begrenzt verdrehbar.

Durch diese Maßnahme wird vorteilhafterweise ein weiterer Freiheitsgrad der Relativbewegung des erfindungsgemäßen Riegels gebildet.

In einer bevorzugten Ausgestaltung weist das erste Eingriffsmittel zumindest eine im wesentlichen quer zur Riegelebene und quer zur Längsrichtung orientierte Fläche auf, die von einer im wesentlichen quer zur Riegelebene und quer zur Längsrichtung orientierten Fläche des zweiten Eingriffsmittels hintergriffen wird, und liegen die Flächen mit einem Spiel aneinander.

Die beiden sich gegenseitig hintergreifenden Flächen an den beiden Eingriffsmitteln bewirken, daß die beiden Teile des erfindungsgemäßen Riegels in Längsrichtung und in Richtung quer zur Riegelebene aneinander gegen Zugkräfte gesichert sind. Auf diese Weise wird vorteilhaft eine mechanisch besonders einfache Verbindung der beiden Teile des Riegels geschaffen. Die Orientierung der beiden Flächen kann dabei senkrecht zur Riegelebene und senkrecht zur Längsrichtung sein, wobei dann noch zwei weitere übereinanderliegende Anlageflächen erforderlich sind, die eine Orientierung etwa parallel zur Riegelebene aufweisen, bspw. in Form von als Hinterschneidungen ausgebildeten Eingriffsmitteln, um die beiden Teile in Richtung quer zur Riegelebene aneinander zu sichern. Vorteilhafter ist es jedoch, wenn die beiden Flächen so orientiert sind, daß sie sowohl eine Komponente senkrecht zur Riegelebene als auch eine Komponente parallel zur Riegelebene aufweisen, so daß gleichzeitig eine Sicherung der Teile in Längsrichtung und in Richtung quer zur Riegelebene gewährleistet ist. Dadurch, daß die Flächen mit einem Spiel aneinander liegen, werden auf mechanisch vorteilhaft einfache Weise Freiheitsgrade eines Gelenks mit begrenzten Drehwinkeln geschaffen.

Weiterhin ist es bevorzugt, wenn eine Stirnseite des einen Teils in der Riegelebene konvex und eine dieser gegenüberliegende Stirnseite des anderen Teils entsprechend konkav ausgebildet ist.

Durch diese Ausgestaltung der beiden sich gegenüberliegenden Stirnseiten der miteinander verbundenen Teile des erfindungsgemäßen Riegels wird vermieden, daß sich die beiden Teile bei einer Relativbewegung in einer Schwenkstellung gegenseitig verklemmen. Außerdem ermöglicht es eine derartige Ausgestaltung der Stirnseiten, die beiden Teile nicht nur in einer Linie hintereinander, sondern je nach den anatomischen Gegebenheiten, bereits in der Riegelebene in einer Winkellage zueinander am Becken zu befestigen.

Bei einem bevorzugten Ausführungsbeispiel ist das erste Eingriffsmittel ein an einem Ende des einen Teils ausgebildeter Vorsprung und das zweite Eingriffsmittel eine an einem Ende des anderen Teils ausgebildete Ausnehmung, wobei der Vorsprung mit Spiel in die Ausnehmung eingreift.

Durch diese Ausgestaltung der beiden Eingriffsmittel der Teile des erfindungsgemäßen Riegels wird eine mechanisch vorteilhaft einfache Verbindung der beiden Teile aneinander gebildet.

Dabei ist es weiter bevorzugt, wenn der Vorsprung einen etwa T-förmigen Querschnitt und die Ausnehmung einen etwa C-förmigen Querschnitt aufweist.

Dies stellt eine konstruktiv vorteilhafte einfache Bauweise dar, mit der die beiden Teile gegen in Längsrichtung und in Querrichtung wirkende Zugkräfte aneinander gesichert und andererseits eine gelenkige Bewegung der beiden Teile relativ zueinander gewährleistet werden kann.

Dabei ist es bevorzugt, wenn sich ein vorderer Abschnitt des Vorsprunges in der Riegelebene quer zur Längsrichtung erstreckt.

Da der Riegel allgemein eine größere Breite als Dicke aufweist, hat diese Maßnahme den Vorteil, daß sich der vordere Abschnitt, der den Querflansch des T-förmigen Querschnitt bildet, über die gesamte Breite des Riegels erstrecken läßt und somit sehr stabil ausgebildet werden kann.

Weiterhin ist es bevorzugt, wenn die Ausnehmung seitlich offen ist.

Die seitlich offene Ausbildung der Ausnehmung hat den Vorteil, daß das andere Teil des erfindungsgemäßen Riegels mit seinem T-förmigen Vorsprung seitlich in die Ausnehmung eingeschoben werden kann. Im nicht am Becken montierten Zustand kann der erfindungsgemäße Riegel somit in seine beiden Teile zerlegt werden. Da derartige Riegel mehrfach verwendet werden, wird dadurch der weitere Vorteil erzielt, daß sich die beiden Teile des erfindungsgemäßen Riegels gut reinigen, bspw. autoklavieren lassen.

In Verbindung mit der gewölbten Ausgestaltung der Stirnseiten der beiden Teile 'wird durch die Ausnehmung, die sich bevorzugt über die gesamte Breite des einen Teils erstreckt, der Vorteil erzielt, daß, wie zuvor erwähnt, die beiden Teile bereits in einer vorgegebenen Winkellage um die quer zur Längsrichtung und quer zur Riegelebene verlaufende Schwenkachse an den Beckenknochen-Abschnitten befestigt werden können. Die Relativbewegung der Teile um die Schwenkachse bzw. Schwenkachsen findet dann um diese durch die Montage am Becken vorgegebene Winkellage statt. Diese Winkellage bei der Montage kann somit in einem relativ weiten Winkelbereich eingestellt werden, wodurch eine bessere Anpassung des erfindungsgemäßen Riegels an die anatomischen Gegebenheiten bzw. den Frakturverlauf möglich ist. Im am Becken montierten Zustand sind die beiden Teile selbstverständlich nicht mehr zerlegbar.

In einer weiteren bevorzugten Ausgestaltung ist die Ausnehmung an einer Rückseite konvex ausgebildet.

Durch diese Maßnahme wird ebenfalls erreicht, daß der in die Ausnehmung eingreifende T-förmig ausgebildete Vorsprung in der Ausnehmung nicht verklemmen kann, wodurch die Funktionssicherheit des erfindungsgemäßen Riegels vorteilhaft verbessert wird.

Bei einem weiteren Ausführungsbeispiel sind das erste Eingriffsmittel und das zweite Eingriffsmittel schwalbenschwanzartig ausgebildet.

Bei dieser Ausgestaltung der beiden Eingriffsmittel wird der Vorteil erzielt, daß die beiden Teile des erfindungsgemäßen Riegels im Bereich der Eingriffsmittel stärker und somit mechanisch belastbarer ausgebildet werden können. Ein weiterer Vorteil dieser Ausgestaltung der Eingriffsmittel besteht darin, daß die beiden Teile identisch ausgebildet werden können, wodurch sich der erfindungsgemäße Riegel kostengünstiger herstellen läßt.

In einer weiteren bevorzugten Ausgestaltung ist der Riegel aus Titan gefertigt.

Durch diese Maßnahme wird ein vorteilhaft gewichtsarmer Riegel geschaffen, der gleichzeitig eine besonders hohe Zug-, Druckund Bruchfestigkeit aufweist. Dabei ist es bevorzugt, wenn der Riegel, d.h. die beiden Teile des Riegels, als Gußstücke hergestellt werden, wobei dann die Eingriffsmittel in einem Stück mit den Teilen des erfindungsgemäßen Riegels ausgebildet werden können.

Weitere Vorteile ergeben sich aus der nachfolgenden Beschreibung der beigefügten Zeichnung.

Es versteht sich, daß die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Ausführungsbeispiele der Erfindung sind in der Zeichnung dargestellt und werden hiernach mit Bezug auf die Zeichnung näher beschrieben. Es zeigen:
- Fig. 1: ein Becken in einer Ansicht von hinten mit einem an dem Becken befestigten Riegel zur Verbindung einer Fraktur des Beckens im Bereich der Schambeinfuge;
- Fig. 2: einen Längsschnitt durch den Riegel in Fig. 1 in der Riegelebene entlang der Linie II-II in Fig. 4;
- Fig. 3: einen Querschnitt des Riegels entlang der Linie III-III in Fig. 2;
- Fig. 4: einen Längsschnitt durch den Riegel entlang der Linie IV-IV in Fig. 2;
- Fig. 5: ausschnittsweise ein zweites Ausführungsbeispiel eines Riegels in einem der Fig. 4 entsprechenden Längsschnitt; und
- Fig. 6: eine Draufsicht auf ein Teil des Riegels in Fig. 5.

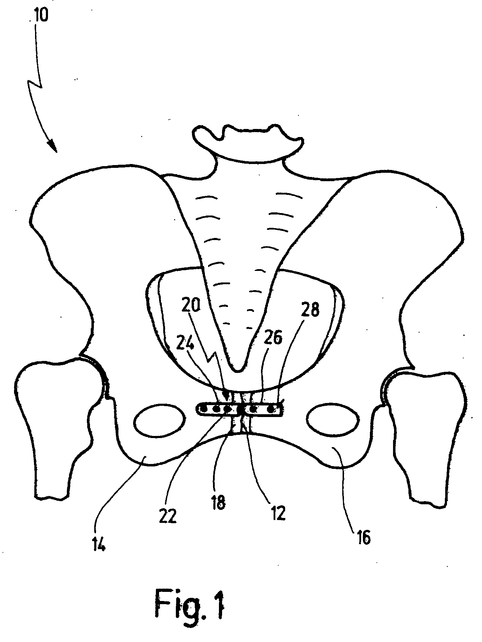

In Fig. 1 ist ein Becken 10 des menschlichen Skeletts dargestellt. In dem Becken 10 ist eine Fraktur 12 vorhanden, die durch äußere Krafteinwirkung, bspw. bei einem Unfall, verursacht wurde. Links der Fraktur 12 befindet sich ein Beckenknochen-Abschnitt 14, der linke Schambogen. Rechts von der Fraktur 12 befindet sich ein weiterer Beckenknochen-Abschnitt 16, der rechte Schambogen. Der linke Beckenknochen-Abschnitt 14 und der rechte Beckenknochen-Abschnitt 16 sind über die Schambeinfuge 18, einem Knorpel, miteinander verbunden. Die Schambeinfuge 18 erlaubt eine Relativbewegung der Beckenknochen-Abschnitte 14 und 16, und zwar in der Art eines Gelenkes mit begrenzten Freiheitsgraden. So können die Beckenknochenabschnitte 14 und 16 sich um die Schambeinfuge 18 als Schwenkachse gegeneinander verschwenken, sich in vertikaler Richtung gegeneinander verschieben oder auch um eine quer durch die Schambeinfuge verlaufende gedachte Schwenkachse verschwenken.

Die Fraktur 12 besteht in dem in Fig. 1 dargestellten Fall in einem Riß in der Schambeinfuge 18, der vertikal in der Schambeinfuge 18 verläuft.

Zur Verbindung der Fraktur 12 ist ein Riegel 20 an den Beckenknochen-Abschnitten 14 und 16 mittels Schrauben oder Nägeln 22 quer zur Fraktur 12 verlaufend befestigt. Die Schrauben bzw. Nägel 22 sind unmittelbar in die Beckenknochen-Abschnitte 14 und 16 eingeschraubt bzw. eingeschlagen.

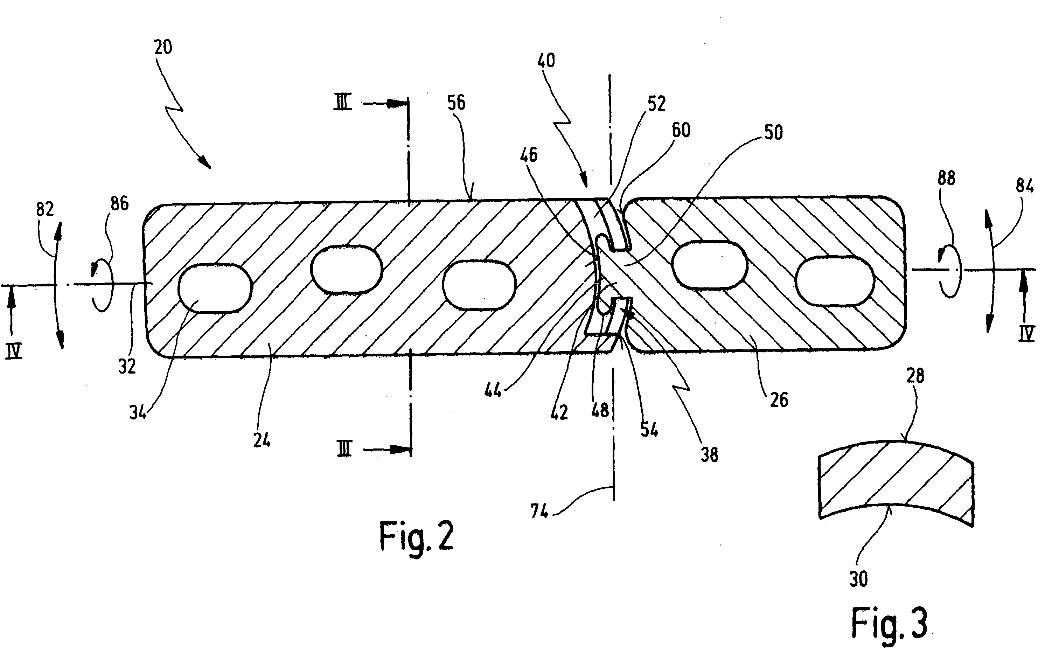

Mit Bezug auf die Fig. 2 bis 4 wird der Riegel 20 nun näher beschrieben.

Der Riegel 20 wird aus zwei Teilen 24 und 26 gebildet. Beide Teile 24 und 26 weisen die Form von plattenförmigen Elementen auf. Ferner sind die beiden Teile 24 und 26 des Riegels 20 aus Titan, bspw. als Gußteile, gefertigt. Eine Oberseite 28 und eine Unterseite 30 der Teile 24 und 26 sind konvex bzw. konkav gewölbt ausgebildet. Mit ihrer jeweiligen Unterseite 30 kommen die Teile 24 und 26 auf dem Becken 10 zu liegen. Je nach den anatomischen Gegebenheiten können die beiden Teile 24 und 26 jedoch auch anders gestaltete Ober- bzw. Unterseiten aufweisen.

Das Teil 24 des Riegels 20 ist bei diesem Ausführungsbeispiel länger als das Teil 26 des Riegels 20, was hier jedoch nur als eine mögliche Wahl der Abmessungen der Teile 24 und 26 zu verstehen ist, die von den anatomischen Gegebenheiten abhängt.

Die beiden Teile 24 und 26 des Riegels 20 sind in Längsrichtung des Riegels 20, d.h. in Richtung einer Längsachse 32, hintereinander angeordnet und miteinander verbunden. Die Verbindungsstelle der beiden Teile 24 und 26 befindet sich im am Becken 10 befestigten Zustand des Riegels 20 etwa über der Fraktur 12.

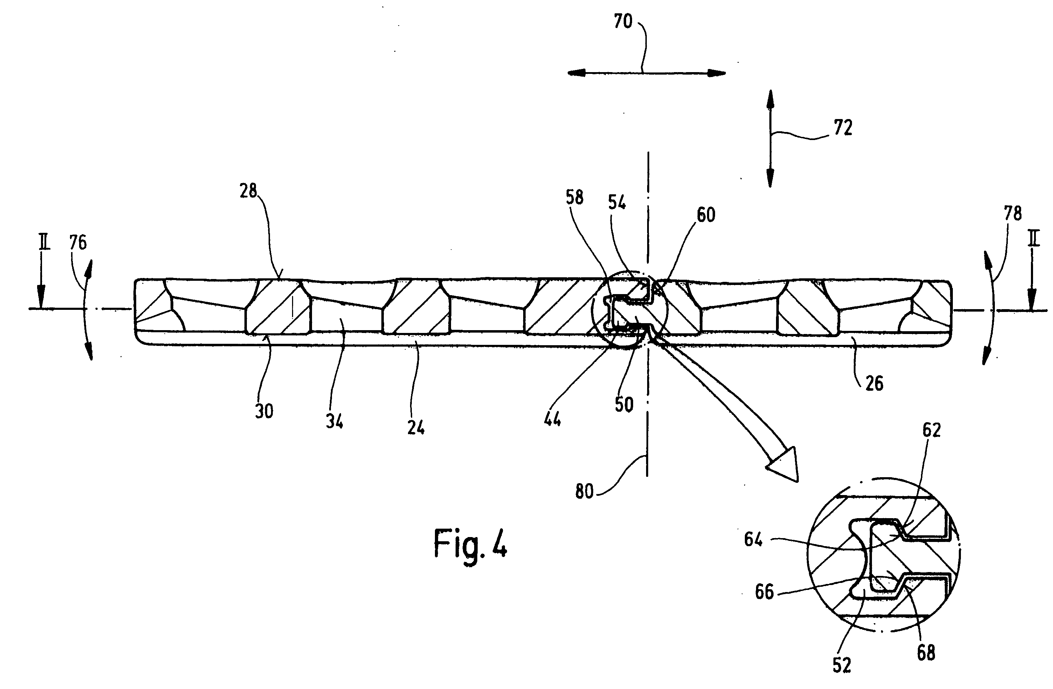

In beiden Teilen 24 und 26 des Riegels 20 sind Durchbohrungen 34 in Form von Langlöchern ausgebildet, in die die Schrauben bzw. Nägel 22 zur Befestigung der Teile 24 und 26 an den Beckenknochen-Abschnitten 14 und 16 durchgeführt werden. In dem Teil 24 sind drei Durchbohrungen 34 und in dem Teil 26 sind zwei Durchbohrungen 34 vorhanden. Wie aus Fig. 4 hervorgeht, erweitern sich die Durchbohrungen 34 zur Oberseite 28 hin, so daß die Köpfe der Schrauben bzw. Nägel 22 in den sich erweiternden Abschnitten der Durchbohrungen 34 versenkt werden können.

Zur Verbindung des Teiles 24 mit dem Teil 26 ist an dem Teil 26 ein erstes Eingriffsmittel 38 vorgesehen, das mit einem an dem Teil 24 vorgesehenen zweiten Eingriffsmittel 40 in Eingriff steht. Das erste Eingriffsmittel 38 ist ein Vorsprung 42 mit T-förmigem Querschnitt. Der Vorsprung 42 ist mit dem Teil 26 einstückig ausgebildet. Ein vorderer Abschnitt 44 des Vorsprunges 42 erstreckt sich in der Riegelebene in Fig. 2 quer zur Längsrichtung des Riegels 20, d.h. quer zur Längsachse 32. Eine Vorderseite 46 des vorderen Abschnittes 44 ist geringfügig konkav gewölbt. Seitliche Enden 48 des vorderen Abschnittes 44 sind abgerundet. Der vordere Abschnitt 44 ist mit dem übrigen Körper des Teiles 26 über einen sich in Längsrichtung des Riegels 20 erstreckenden Steg 50 verbunden.

Das zweite Eingriffsmittel 40 des Teiles 24 ist eine Ausnehmung 52 mit C-förmigem Querschnitt. An einer Stirnseite 54 ist die Ausnehmung 52 in Form eines Schlitzes offen, der quer zur Riegelebene eine geringere lichte Weite aufweist als der übrige Hohlraum der Ausnehmung 52. Ebenso ist die Ausnehmung 52 an einer Seite 56 des Teiles 24 offen. Eine Rückseite 58 der Ausnehmung 52 ist konvex in Form einer Wulst ausgebildet.

Wenn die Teile 24 und 26 des Riegels 20 nicht an dem Becken 10 befestigt sind, läßt sich das Teil 26 von dem Teil 24 trennen, indem der Vorsprung 42 des Teiles 26 aus der Ausnehmung 52 seitlich herausgeführt wird. Umgekehrt werden die Teile 24 und 26 miteinander verbunden, indem der Vorsprung 42 seitlich in die Ausnehmung 52 eingeschoben wird.

Die Stirnseite 54 des Teiles 24 ist in der Riegelebene konvex ausgebildet. Entsprechend ist eine der Stirnseite 54 des Teiles 24 gegenüberliegende Stirnseite 60 des Teiles 26 entsprechend konkav ausgebildet. Auch die Ausnehmung 52 und der vordere Abschnitt 44 des Vorsprunges 42 sind in der Riegelebene gewölbt ausgebildet.

Die Ausnehmung 62 weist eine quer zur Riegelebene und quer zur Längsrichtung orientierte Fläche 62 auf, die hinter eine quer zur Riegelebene und quer zur Längsrichtung orientierte Fläche 64 des vorderen Abschnittes 44 des Vorsprunges 42 greift. Das gleiche gilt für eine Fläche 66 des Vorsprunges 42, die hinter eine Fläche 68 der Ausnehmung 52 greift. Durch die sich gegenseitig hintergreifenden Flächen 62 und 64 bzw. 66 und 68 sind die beiden Teile 24 und 26 des Riegels 20 in Richtung eines Doppelpfeiles 70, d.h. in Längsrichtung des Riegels 20, aneinander gesichert. Dadurch, daß der Vorsprung 42 in der Ausnehmung 52 teilweise eingefaßt aufgenommen ist, sind die Teile 24 und 26 auch in Richtung eines Doppelpfeiles 72, d.h. quer zur Riegelebene, aneinander gesichert. Die Flächen 62 und 64 bzw. 66 und 68 liegen mit einem Spiel aneinander.

Der Vorsprung 42 ist in der Ausnehmung 52 insgesamt mit einem Spiel aufgenommen, das eine gelenkige Bewegung der Teile 24 und 26 zueinander in einem begrenzten Winkelbereich zuläßt. In Fig. 2 ist eine in der Riegelebene liegende und quer zur Längsrichtung des Riegels 20 verlaufende Schwenkachse 74 eingezeichnet, um die die Teile 24 und 26 des Riegels 20 gemäß Doppelpfeilen 76 und 78 begrenzt gegeneinander verschwenkt werden können. Die Schwenkbewegung um die Schwenkachse 74 ist in einem Winkelbereich von etwa 0 bis 5° möglich. Die Schwenkbewegung ist dadurch begrenzt, daß die Stirnseiten 54 und 60 bei einer bestimmten Verschwenkung aneinanderstoßen und somit die Schwenkbewegung begrenzen. Die Schwenkbewegung ist jedoch auch durch das Spiel des Vorsprunges 42 in der Ausnehmung 52 begrenzt.

Ferner ist in Fig. 4 eine quer zur Riegelebene und quer zur Längsrichtung des Riegels 20 verlaufende Schwenkachse 80 eingezeichnet, um die die beiden Teile 24 und 26 gemäß Doppelpfeilen 82 und 84 in Fig. 2 relativ zueinander verschwenkbar sind, wobei die Schwenkbewegung um die Schwenkachse 80 wiederum durch das Aneinanderstoßen der Stirnseiten 54 und 60 der Teile 24 und 26 begrenzt ist. Die Schwenkbewegung um die Schwenkachse 80 ist ebenfalls in einem Winkelbereich von 0 bis 5° möglich.

Während die Teile 24 und 26 in Fig. 1 in einer Linie hintereinander angeordnet sind, ist es auch möglich, die Teile 24 und 26, falls erforderlich, bereits in einer bestimmten Winkellage relativ zueinander um die Schwenkachse 80 verschwenkt am Becken 10 zu befestigen. Diese Winkellage kann dadurch eingestellt werden, daß der Vorsprung 42 über die in Fig. 2 dargestellte Mittellage hinaus in die Ausdehnung 52 oder in eine Position vor der Mittellage eingeschoben wird.

Schließlich läßt das Spiel des Vorsprunges 42 in der Ausnehmung 52 eine gegensinnige Verdrehung der Teile 24 und 26 um die Längsachse 32 in einem begrenzten Winkelbereich von etwa 0 bis 5° zu, wie mit Pfeilen 86 und 88 in Fig. 2 angedeutet ist.

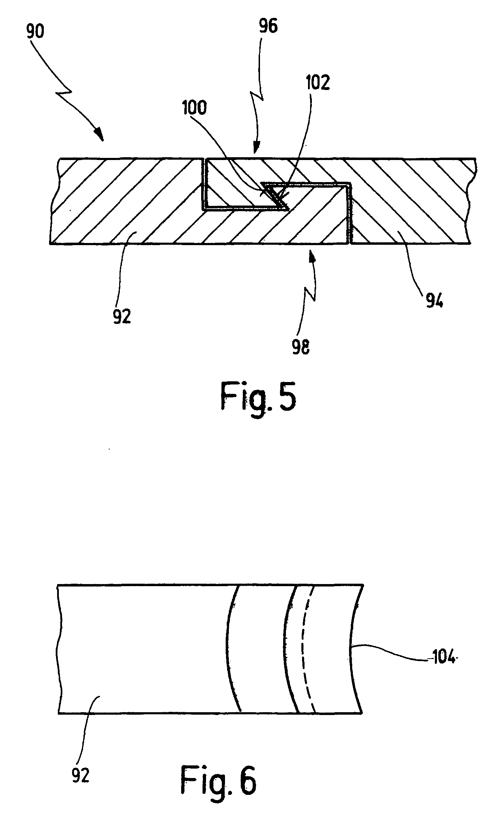

In Fig. 5 und 6 ist ein zweites Ausführungsbeispiel eines Riegels 90 dargestellt, der aus einem Teil 92 und einem Teil 94 gebildet ist. Die Teile 92 und 94 sind identisch.

Das Teil 94 des Riegels 90 weist ein erstes Eingriffsmittel 96 auf, das mit einem zweiten Eingriffsmittel 98 des Teiles 92 in Eingriff steht.

Das erste Eingriffsmittel 96 und das zweite Eingriffsmittel 98 bilden eine schwalbenschwanzartige Verbindung der beiden Teile 92 und 94. Das erste Eingriffsmittel 96 weist eine quer zur Längsrichtung des Riegels 90 und quer zur Riegelebene orientierte Fläche 100 auf, die eine in etwa gleich orientierte Fläche 102 des zweiten Eingriffsmittels 98 des Teiles 92 hintergreift. Die Flächen 100 und 102 sind durch an den Teilen 92 und 94 des Riegels 90 ausgebildete Hinterschneidungen gebildet. Die Flächen 100 und 102 sichern die Teile 92 und 94 sowohl in Längsrichtung des Riegels 90 als auch in Richtung quer zur Riegelebene aneinander. Auch bei diesem Ausführungsbeispiel weisen jeweils aneinanderliegende Flächen des Teiles 92 und des Teiles 94 ein Spiel auf, um die Teile 92 und 94 mit einer begrenzten Relativbeweglichkeit miteinander zu verbinden. Ebenso ist eine Stirnseite 104 des Teiles 92 konkav gewölbt ausgebildet, wobei die der Stirnseite 104 gegenüberliegende entsprechende Stirnseite des Teiles 94 konvex ausgebildet ist.

Im übrigen entspricht der Riegel 90 in seiner Funktion dem Riegel 20.

## Patentansprüche

1. Riegel zur Verbindung einer Fraktur (12) des Beckens (10) im Bereich der Schambeinfuge, der beidseits der Fraktur (12) an den zu verbindenden Beckenknochen-Abschnitten (14, 16) mittels Schrauben, Nägeln (22) oder dgl. befestigbar ist, **dadurch gekennzeichnet, daß** der Riegel (20; 90) aus zumindest zwei in Längsrichtung hintereinander angeordneten Teilen (24, 26; 92, 94) gebildet ist, wobei zur Verbindung der Teile (24, 26; 92, 94) an dem einen Teil (26; 94) ein erstes Eingriffsmittel (38; 96) vorgesehen ist, das mit einem an dem anderen Teil (24; 92) vorgesehenen zweiten Eingriffsmittel (40; 98) in Eingriff steht, derart, daß die beiden Teile (24, 26; 92, 94) in Längsrichtung und in Richtung quer zur Riegelebene aneinander gesichert sind, und daß die beiden Teile (24, 26; 92, 94) relativ zueinander eine begrenzte gelenkige Beweglichkeit aufweisen.

2. Riegel nach Anspruch 1, **dadurch gekennzeichnet, daß** die Teile (24, 26; 92, 94) eine begrenzte gelenkige Beweglichkeit um eine quer zur Riegelebene verlaufende erste Schwenkachse (80) aufweisen.

3. Riegel nach Anspruch 1 oder 2, **dadurch gekennzeichnet, daß** die Teile (24, 26; 92, 94) eine begrenzte gelenkige Beweglichkeit um eine in der Riegelebene quer zur Längsrichtung verlaufende zweite Schwenkachse (74) aufweisen.

4. Riegel nach einem der Ansprüche 1 bis 3, **dadurch gekennzeichnet, daß** die beiden Teile (24, 26; 92, 94) relativ zueinander um ihre Längsachse (32) gegensinnig um einen begrenzten Winkel verdrehbar sind.

5. Riegel nach einem der Ansprüche 1 bis 4, **dadurch gekennzeichnet, daß** das erste Eingriffsmittel (38; 96) zumindest eine im wesentlichen quer zur Riegelebene und quer zur Längsrichtung orientierte Fläche (60, 66; 102) aufweist, die von einer im wesentlichen quer zur Riegelebene und quer zur Längsrichtung orientierten Fläche (62, 68; 100) des zweiten Eingriffsmittels (40; 98) hintergriffen wird, und daß die Flächen (62, 64, 66, 68; 100, 102) mit einem Spiel aneinanderliegen.

6. Riegel nach einem der Ansprüche 1 bis 5, **dadurch gekennzeichnet, daß** eine Stirnseite (54; 104) des einen Teils (24; 92) konvex und eine dieser gegenüberliegende Stirnseite (60) des anderen Teils (26; 94) konkav ausgebildet ist.

7. Riegel nach einem der Ansprüche 1 bis 6, **dadurch gekennzeichnet, daß** das erste Eingriffsmittel (38) ein an einem Ende des einen Teils (26) ausgebildeter Vorsprung (42) und das zweite Eingriffsmittel (40) eine an einem Ende des anderen Teils (24) ausgebildete Ausnehmung ist, wobei der Vorsprung (42) mit Spiel in die Ausnehmung (52) eingreift.

8. Riegel nach Anspruch 7, **dadurch gekennzeichnet, daß** der Vorsprung (42) einen etwa T-förmigen Querschnitt und die Ausnehmung (52) einen etwa C-förmigen Querschnitt aufweist.

9. Riegel nach Anspruch 8, **dadurch gekennzeichnet, daß** sich ein vorderer Abschnitt (44) des Vorsprungs (42) in der Riegelebene quer zur Längsrichtung erstreckt.

10. Riegel nach einem der Ansprüche 7 bis 9, **dadurch gekennzeichnet, daß** die Ausnehmung (52) seitlich offen ist.

11. Riegel nach einem der Ansprüche 7 bis 10, **dadurch gekennzeichnet, daß** die Ausnehmung (52) an einer Rückseite (58) konvex ausgebildet ist.

12. Riegel nach einem der Ansprüche 1 bis 6, **dadurch gekennzeichnet, daß** das erste Eingriffsmittel (96) und das zweite Eingriffsmittel (98) schwalbenschwanzartig ineinandergreifen.

13. Riegel nach einem der Ansprüche 1 bis 12, **dadurch gekennzeichnet, daß** er aus Titan gefertigt ist.

## Claims

1. A plate for joining a fracture (12) of the pelvis (10) in the region of the pubic symphysis, the plate being fastenable on either side of the fracture by means of screws, nails (22) or the like to the pelvic bone portions (14, 16) to be connected, **characterized in that** the plate (20; 90) is formed of at least two parts (24, 26; 92, 94) arranged along a longitudinal direction one behind the other, wherein first engaging means (38; 96) are provided on one part (26; 94) for connecting the parts (24, 26; 92, 94), which engage with second engaging means (40; 98) provided on the other part (24; 92), such that the two parts (24, 26; 92, 94) are secured to one another in longitudinal direction and in a direction transverse to the plate plane, and the two parts (24, 26; 92, 94) have a limited articulated movability relative to one another.

2. The plate of claim 1, **characterized in that** the parts (24, 26; 92, 94) have a limited articulated movability about a first pivot axis (80) running transversely to the plate plane.

3. The plate of claim 1 or 2, **characterized in that** the parts (24, 26; 92, 94) have a limited articulated movability about a second pivot axis (74) running in the plate plane transversely to the longitudinal direction.

4. The plate of anyone of claims 1 through 3, **characterized in that** the two parts (24, 26; 92, 94) are counter-directionally rotatable with respect to one another about their longitudinal axis (32) by a limited angle.

5. The plate of anyone of claims 1 through 4, **characterized in that** the first engaging means (38; 96) comprise at least one surface (60, 66; 102) oriented substantially transversely to the plate plane and transversely to the longitudinal direction, which is engaged behind by a surface (62, 68; 100) of the second engaging means (40; 98) oriented substantially transversely to the plate plane and transversely to the longitudinal direction, and the surfaces (62, 64, 66, 68; 100, 102) adjoin with play therebetween.

6. The plate of anyone of claims 1 through 5, **characterized in that** an end face (54; 104) of one part (24; 92) is formed to be convex and an opposing end face (60) of the other part (26; 94) is formed to be concave.

7. The plate of anyone of claims 1 through 6, **characterized in that** the first engaging means (38) are a projection (42) formed at one end of the one part (26) and the second engaging means (40) are a recess (52) formed at one end of the other part (24), wherein the projection (42) engages the recess (52) with play therebetween.

8. The plate of claim 7, **characterized in that** the projection (42) has a substantially T-shaped cross section and the recess (52) has a substantially C-shaped cross section.

9. The plate of claim 8, **characterized in that** a front portion (44) of the projection (42) extends in the plate plane transversely to the longitudinal direction.

10. The plate of anyone of claims 7 through 9, **characterized in that** the recess (52) is open to the side.

11. The plate of anyone of claims 7 through 10, **characterized in that** the recess (52) is formed to be convex at a backside (58).

12. The plate of anyone of claims 1 through 6, **characterized in that** the first engaging means (96) engage with the second engaging means (98) in dovetail manner.

13. The plate of anyone of claims 1 through 12, **characterized in that** the plate is made of titanium.

## Revendications

1. Plaque pour assembler les parties d'une fracture (12) du bassin (10) dans la zone de la symphise pubienne, susceptible d'être fixée de part et d'autre de la fracture (12) sur les parties des os du bassin (14, 16) qu'il convient d'assembler, au moyen de vis, de clous (22) ou similaires, **caractérisée en ce que** la plaque (20 ; 90) est constituée d'au moins deux parties (24, 26 ; 92, 94) disposées l'une derrière l'autre dans la direction longitudinale, un premier moyen d'engagement (38 ; 96) étant prévu sur l'une des parties (26 ; 94), pour assembler les parties (24, 26 ; 92, 94), moyen qui coopère avec un deuxième moyen d'engagement (40 ; 98) prévu sur l'autre partie (24 ; 92), de telle sorte que les deux parties (24, 26 ; 92, 94) soient maintenues l'une contre l'autre dans la direction longitudinale et dans la direction perpendiculaire au plan de la plaque, et **en ce que** les deux parties (24, 26 ; 92, 94) sont articulées l'une à l'autre avec une mobilité limitée.

2. Plaque selon la revendication 1, **caractérisée en ce que** les parties (24, 26 ; 92, 94) sont articulées avec une mobilité limitée autour d'un premier axe de pivotement (80) perpendiculaire au plan de la plaque.

3. Plaque selon la revendication 1 ou 2, **caractérisée en ce que** les parties (24, 26 ; 92, 94) sont articulées avec une mobilité limitée autour d'un deuxième axe de pivotement (74) s'étendant, dans le plan de la plaque, perpendiculairement à la direction longitudinale.

4. Plaque selon l'une des revendications 1 à 3, **caractérisée en ce que** les deux parties (24, 26 ; 92, 94) peuvent tourner l'une par rapport à l'autre autour de leur axe longitudinal (32), dans le sens inverse des aiguilles d'une montre, selon un angle limité.

5. Plaque selon l'une des revendications 1 à 4, **caractérisée en ce que** le premier moyen d'engagement (38 ; 96) présente au moins une surface (60, 66 ; 102) orientée de façon sensiblement perpendiculaire au plan de la plaque et perpendiculaire à la direction longitudinale, qui est saisie par derrière par une surface (62, 68 ; 100) du deuxième moyen d'engagement (40 ; 98) qui est orientée de façon sensiblement perpendiculaire au plan de la plaque et perpendiculaire à la direction longitudinale, et **en ce que** les surfaces (62, 64, 66, 68 ; 100, 102) sont placées l'une contre l'autre avec un jeu.

6. Plaque selon l'une des revendications 1 à 5, **caractérisée en ce qu'**une face frontale (54 ; 104) de l'une des parties (24 ; 92) est convexe et une face frontale (60) de l'autre partie (26 ; 94), située en regard de celle-ci, est concave.

7. Plaque selon l'une des revendications 1 à 6, **caractérisée en ce que** le premier moyen d'engagement (38) est une saillie (42) formée à une extrémité de l'une des parties (26) et le deuxième moyen d'engagement (40) est un évidement ménagé à une extrémité de l'autre partie (24), la saillie (42) pénétrant avec jeu dans l'évidement (52).

8. Plaque selon la revendication 7, **caractérisée en ce que** la saillie (42) présente une section transversale approximativement en forme de T et l'évidement (52) présente une section transversale approximativement en forme de C.

9. Plaque selon la revendication 8, **caractérisée en ce qu'**un tronçon avant (44) de la saillie (42) s'étend dans le plan de la plaque, perpendiculairement à la direction longitudinale.

10. Plaque selon l'une des revendications 7 à 9, **caractérisée en ce que** l'évidement (52) est ouvert sur le côté.

11. Plaque selon l'une des revendications 7 à 10, **caractérisée en ce que** l'évidement (52) est convexe sur une face arrière (58).

12. Plaque selon l'une des revendications 1 à 6, **caractérisée en ce que** le premier moyen d'engagement (96) et le deuxième moyen d'engagement (98) pénètrent l'un dans l'autre à la façon d'une queue d'aronde.

13. Plaque selon l'une des revendications 1 à 12, **caractérisée en ce qu'**elle est constituée en titane.
